# EUROPEAN PATENT APPLICATION

(11) **EP 0 900 807 A1**
(43) Date of publication of application: **10.03.1999**
(21) Application number: 97202735.3
(22) Date of filing: 05.09.1997
(51) Int. Cl.: C08B 31/00, A61L 15/00

(54) **Absorbing material based on starch having improved absorbent properties and process for the preparation thereof**

(71) Applicant: INSTITUUT VOOR AGROTECHNOLOGISCH ONDERZOEK (ATO-DLO), 6700 AA Wageningen (NL)
(72) Inventor: Feil, Herman, 6717 RG Ede (NL)
(74) Representative: de Bruijn, Leendert C.

(57) **Abstract**

A biodegradable, highly water-absorbing polymer based on starch or derivatives thereof is described, wherein the starch has not been chemically modified or only to a degree of substitution below 0.2, and has a water-absorbing power of at least 10 times its own weight, half of the water absorption being attained within 3 minutes.

A process for producing such a water-absorbing polymer is also described, the process comprising modifying and treating the starch in a co-continuous water-oil or oil-water system in such a way that an open, slightly crosslinked structure is fixated.

## Description

The invention relates to starch materials having a high water-absorbing power and a fast water absorption, which are readily biodegradable and which are based on native starch or starch which is only slightly modified.

It is known (Aggour and Mansoura, Starch/Stärke 45 (1993), pages 55-59) to produce hydrogels on the basis of starch by dissolving starch in an alkaline solution, followed by addition of acrylamide and a crosslinking agent (N,N'-methylene bisacryl-amide). The maximum water absorption of such modified starch-acrylamide is 140 g/g.

According to Kulicke et al. (Starch/Stärke 41 (1989), pages 140-146) a water- absorbing modified starch can be prepared by crosslinking starch, oxidised starch or amylopectin with 25 wt.% of epichlorohydrin. The maximum water absorption is 50 g/g and is attained after several hours.

A water-absorbent carboxymethyl starch with a DS (degree of substitution) of 0.6-0.9 produced by extrusion is disclosed in NL-A-9100249. Self-crosslinking of carboxymethyl starch (DS 0.5-1.25) by drying at high temperature is described in US-A-5079354. EP-A-637594 discloses carboxymethyl cellulose (DS 0.3-0.6) cross-linked with aminoacids as a water-absorbent resin. GB-A-1576475 teaches gelatinising starch and, during or after gelatinisation, crosslinking with epichlorohydrin or the like to a DS of 0.003-0.02 and carboxymethylating to a DS of at least 0.2 (0.25-0.71).

Although such known hydrogels based on starch sometimes exhibit an acceptable water-absorbing capacity, the rate of water absorption is often too slow for most applications. Another great disadvantage of these known absorbing starch derivatives is that they are highly modified starches, or copolymers of starch and synthetic polymers, consisting for 25-50 wt.% of non-natural and poorly degradable structures, such as polyacrylamide, polyacrylic acid and polyhydroxypropylene. The presence of residual monomers cause these products also to have some toxicity. Therefore, such products are environmentally harmful and suspect from the health point of view.

Starch itself has a number of properties which limit the absorption of water. The predominant limitation results from the strong internal interactions of starch by hydrogen bridging causing starch to be water-insoluble. The starch chains have a stronger tendency, even after gelatinisation or melting of starch, to interact among each other, than to fully hydrate. Only strong modification, in particular with charged groups, can render the chains effectively water binding. The products thus obtained have an unacceptably low degradability, as discussed above, and, moreover, they are expensive. The presence of charge has as an additional disadvantage, that the water absorption is strongly dependent on salt concentrations. Salts and other solutes normally present, such as in urine, lead then to a considerable decrease of swelling.

A water-absorbing polymer based on starch has now been found which does not have the disadvantages mentioned above. In particular, the polymer is characterised by an open structure, wherein a high water-absorbing capacity is coupled to a fast water absorption. Moreover, the products found are fully biodegradable. Furthermore, the water-absorbing capacity hardly depends on the salt concentration.

The invention therefore pertains to a biodegradable, high water-absorbing polymer based on starch or derivatives thereof, of which the starting material has not been chemically modified or has been modified for a maximum degree of substitution, DS, of 0.2 and has a water-absorbing power of at least 10 times, in particular at least 20 times its own weight, half of the maximum water absorption being obtained within 3 minutes, especially within 1 minute. It is to be understood that the maximum DS referred to above refers to the initial starch used for producing the highly absorptive starch of the invention. The maximum DS relates to substituents such as alkyl, carboxyalkyl, hydroxyalkyl, ammonioalkyl and the like, as well as combinations thereof, which substituents may conventionally be used for increasing the absorption capacity of the starch, but which are not desired according to the invention. The maximum DS of 0.2 does not include substituents which are the result of crosslinking reactions, such as phosphono, dihydroxyalkyl, carboxylalkylcarbonyl and the like, and divalently coupled substituents such as phosphinico, hydroxyalkylene and the like. A maximum DS of 0.2 means for derivatives that can contain two derivative function in the same anhydroglucose unit, such as dialdehyde starch, that the percentage of modification is 10%. The polymer according to the invention contains a low charge density of the crosslinks, in particular less than 1 ionic charge per 25 anhydroglucose units, more in particular less than 1 charge per 50 anhydroglucose units. Starch is understood to comprise starch in all its physical modifications.

The invention also provides processes for preparing the polymers according to the invention. According to such a process, starch is crosslinked in a co-continuous phase of water and a hydrophobic solvent, such as an oil or fat. A co-continuous phase is understood to be an emulsion wherein both the aqueous phase and the oil phase are continuous or quasi-continuous, i.e. one of the phases may partly consist of discrete particles, e.g. water in oil. An emulsifier may optionally be used in order to obtain an oil-water-emulsion of the desired composition.

The starch to be used according to the invention may be any native granular starch (potato, corn, wheat, rice, tapioca, etc.), as well as physically or enzymatically modified starch such as pregelatinised starch, maltodextrins, amylase-treated starch and acid-treated starch. The starch may also be a chemically modified starch, such as oxidised or chlorinated starch (hypochlorite-oxidised starch, carboxy starch, dialdehyde starch), starch esters (acetate, succinate, alkenylsuccinate, half-esters, phosphate esters), non-ionic starch ethers such as alkyl and hydroxyalkyl (hydroxyethyl, hydroxypropyl) starch and cyanoethyl starch, cationic starch ethers such as trimethylammonio-alkyl starch or other cationic starches and anionic starch ethers, such as carboxymethyl starch, and furthermore starch resulting from combinations of modifications, i.e. bifunctional starch. Preferably starch is used which is not or only slightly (DS <0.2) chemically modified.

The crosslinking agents may be common bi- or polyfunctional reagents known as crosslinkers. Examples thereof are phosphates such as trisodium trimetaphosphate (TSTP), functional epoxides such as epichlorohydrin, diepoxybutane and other α,ω-diepoxyalkanes, diglycidylether and alkylene bis-glycidyl ethers, dichlorohydrin, dibromohydrin, phosphoryl chloride, phosphorus oxychlorohydrin, adipic anhydride or other polycarboxylic anhydride and borax. TSTP is particularly preferred. Examples of crosslinkers for modified starch are dialdehydes (glyoxal, glutaraldehyde), diamines (urea, hexamethylenediamine) and aminoacids; for example, urea may be used to crosslink dialdehyde starch. The amount of cross-linking agents used, is for example 1-30 units, especially 2-20 units per 100 anhydroglucose units.

The hydrophobic liquid may be a conventional di- or triglyceride (fat or oil) which is liquid at the processing temperature. Examples are paraffin oil, palm oil, palm kernel oil, sunflower oil, butter, oil, vegetable oil, waxes such as bees was, bamboo leaf wax, Japan wax, shellac wax, microcrystalline wax, paraffin wax. A hydrophobic liquid or oil also covers apolar solvents, such as petrol, aliphatic, cycloaliphatic or aromatic hydrocarbons, e.g. cyclohexane, octane, dodecane, toluene, xylene, decaline, higher alcohols such as pentanol, as well as mixtures of these solvents and mixtures thereof with triglyceride oils. The oil is selected according to its viscosity, which should not differ too much from the viscosity of the aqueous starch phase. The volume ratio of oil to water may be between 10:1 and 1:5, especially between 5:1 and 1:4.

Emulsifiers that can be used for preparing the emulsion include fatty acid monoglycerides such as Dimodan, Acidan (distilled monoglycerides) and glycerol monostearate, citric, lactic, diacetyl-tartaric and acetic acid esters of monoglycerides (Cetodan, Lactodan, Panodan, Promodan), propylene glycol esters of fatty acids (Triodan), sorbitan monolaurate, sorbitan monopalmitate, calcium stearate, ethoxylated and succinylated monoglycerides, glucose and sucrose esters; also fatty alcohols (cetanol, stearyl alcohol, palmitol), fatty acids (stearic, palmitic, oleic, linoleic acid), lipids, phospholipids, lecithin, glycolipids, glycols (propylene glycol, polyethylene glycol) can be used as well as commercial emulsifiers (Tween, Span). Emulsifying methods may involve the use of homogenisers, ultrasoner/ultrasonic transducers (e.g. Bransons Sonifier 250, Ultra thurrax emulsifier) and various other mixing systems. The amount of emulsifier can be e.g. 0.01-10, especially 0.1-5 wt.% with respect to the total mixture of starch, water, oil and emulsifier.

The process can be performed by mixing an aqueous solution or suspension of starch (e.g. 4-60 wt.% starch) with the crosslinking agent and the oil phase and stirring the mixture for several minutes to 3 days, especially for 2-36 hours. The reaction conditions are chosen so as to obtain the desired distribution of aqueous phase and oil phase. In particular, the mixing energy and the use of emulsifiers determines the ratios oil-in-water vs. water-in-oil or the presence of two continuous phases. By selecting the appropriate conditions such as mixing energy, also a particulate product is obtained with particle sizes in the range of 100 nm to 1 mm and which usually has a monodisperse size distribution (in particular with a polydispersity below 30%). The reaction temperature may be between 0 and 100 °C, preferably between 20 and 80 °C. If necessary a crosslinking catalyst, such as a base, may be added before or during stirring. Preferably, the starch is gelatinised during crosslinking, but it may also be done before crosslinking if desired. This can be achieved by performing the reaction at about the gelatinisation temperature (50-70 °C), or by subjecting the starch to alkaline conditions.

The absorption capacity of the final product may be increased by allowing the starch to swell by addition of water after crosslinking and before separating the oil phase. If the product is intended for use as a carrier for an active substance, the active substance may be added before or during carrying out the process of the invention or afterwards.

After the crosslinking reaction, the oil phase is removed by extraction with e.g. ethanol, propanol or another alcohol, acetone, ether and the like. The product may be further processed to adjust its desired properties, e.g. by preswelling in water by single or repeated addition of water for several minutes or hours (e.g. 10 min. to 36 hours), washing, grinding, sieving, agglomerating and the like.

The invention pertains both to the processes described above and to the products obtained with these processes.

The polymers thus obtainable can be used in many applications as a result of their biodegradability and their excellent water-absorbing properties:
- sanitary products, such as nappies, sanitary napkins, serviettes;
- medical aids such as bandages and swathes,
- coatings for drugs, in particular for controlling the release rate by degradation of the hydrogel;
- humidity controlling agents for agriculture and horticulture, as well as for hydrocultures and the like;
- encapsulants for colorants, fragrances and perfumes;
- encapsulants for fertilisers and nutrients;
- pet litter material;
- gel filtration columns.

### EXAMPLES

### Example 1

65 g of Paselli SA-2 (Avebe) is dissolved in 250 ml distilled water. To the solution is added 26 g TSTP. After homogenisation, the starch solution is dispersed in 500 ml cyclohexane in a 1000 ml beaker with a stirrer with four wings at 650 rpm. While stirring, 50 ml of a 1.65 M NaOH solution is added to the dispersion. After 16 h, the stirrer is stopped and the crosslinked starch particles are isolated. The size of the particles before drying is approximately 300-400 µm. Before drying, the product had a water absorption capacity of 20 g/g based on dry material within one hour. Part of the product is air-dried immediately after isolation, part is swollen in water for 65 hours, followed by washing three times with ethanol. After drying, the water absorption of the none swollen product is 14 g/g after one hour, as opposed to a water absorption of 22 g/g after one hour for the swollen product. In a 0.9 wt% NaCl solution, the non-swollen particles show a maximum absorption of 11 g/g after two hours, of which 64% is absorbed within the first hour. The swollen particles have a maximum absorption of 12 g/g after one hour, of which 90% is absorbed within the first five minutes.

### Example 2

A solution is prepared of 15 g carboxymethyl starch (DS 0.12) in 175 ml water. After homogenisation, 3.4 g TSTP is added to the solution. When the TSTP is dissolved, the starch solution is dispersed in 200 ml salad oil. Subsequently, a solution of 1.3 g NaOH in 25 ml water is added to the mixture. The mixture is stirred for 17.5 hours at a rate of 650 rpm. After completion of the reaction, the particles are separated from the oil via filtering. Then, 1000 ml water is added to allow the particles to swell, thus creating an open structure. After 48 hours of swelling, the particles are isolated via filtering and washed with ethanol, followed by air drying.
After drying, the product has a water absorption capacity of 25 g/g, of which 91 % is absorbed within the first five minutes. The absorption capacity in a 0.9 wt% NaCl solution is 12 g/g within five minutes.

### Example 3

A solution is prepared of 15 g carboxymethyl starch (DS =0.12) in 175 ml water. After homogenisation, 3.4 g TSTP is added to the solution and dissolved. Meanwhile, 10 g SPAN 65 is dissolved in 400 ml salad oil. The starch solution is dispersed in the oil, while stirring at a rate of 650 rpm. Subsequently, a solution of 1.3 g NaOH in 25 ml water is added to the mixture. After a reaction time of 7 hours, the starch particles are separated from the oil by addition of acetic acid. After 14 hours, the layers are separated in a separating funnel. 500 ml water is added to the product for washing; then, the product is put in water for swelling. After swelling for 48 hours, the product is removed from the water by precipitation with ethanol and dried. It has a water absorption capacity of 26 g/g within one hour, of which 98% is absorbed within the first five minutes. In a 0.9 wt% NaCl solution, the absorption is 12 g/g within one hour, of which 75 % is absorbed within the first five minutes.

### Example 4

10 g PN (granular potato starch; Avebe) is dispersed in 175 ml water. Then, 2 g TSTP is added to the solution and dissolved. The mixture is dispersed in 400 ml paraffin oil while stirring at a rate of 650 rpm. Subsequently, 25 ml of a 2M NaOH solution is added. After 23 hours, 100 ml concentrated acetic acid is added to the reaction mixture, allowing to separate the starch particles from the paraffin oil. The phases are separated in a separating funnel. The starch-containing phase is diluted with 200 ml water to a total volume of 500 ml and precipitated in 1000 ml ethanol. The product is washed twice with ethanol and air-dried. After drying, the water absorption capacity is 89 g/g within two hours, of which 82% is absorbed within the first hour. In a 0.9 wt% NaCl solution, the absorption is 20 g/g within one hour, of which 81% is absorbed within the first five minutes.

### Example 5

66 ml of cyclohexane containing 1.6 g of span 65 was heated to a temperature of 500 °C. 10 g of Paselli (6) was dissolved in 25 ml of water while stirring. To the Paselli solution 2 g of TSTP was added. The two solutions were mixed together into a round bottom flask and emulsified during 5 min using a Branson ultrasonic device. After emulsification 8 ml of 1 M NaOH was added by drops while stirring. Stirring was continued for 20 h at room temperature. The emulsion was mixed with 20 ml of water and 20 ml of acetic acid. The mixture was left standing for 1 h. Thereafter the water phase, containing the starch particles, was separated from the cyclohexane. The particles were washed in demi water by repeated ultracentrifugation. Sizes of the particles were determined with light microscopy and dynamic light scattering. Mean particle diameter was 800 nm with a polydispersity of 30%.

### Example 6

A 10% starch solution was refluxed boiled for several hours 25 ml of the low viscous liquid was emulsified with 66 ml of a warm cyclohexane solution in which 1.6 g Span 65 was dissolved. The two solutions were emulsified using a ultra thurrax emulsifying device. Under continuous stirring 4 ml of a 2 M NaOH was added as drops. The mixture was left stirring for 20 h 20 ml of water and 10 ml of octanoic acid were added and the mixture was vigorously shaken. The mixture was left standing for 1 h. The water phase, containing the starch particles, was separated from the oil phase. The particles were washed in demi water by repeated ultra-centrifugation. Sizes of the particles in water ranged from 0.2 to 10 micron measured with light and electron microscopy.

### Example 7

7.5 g of Carboxymethyl starch (DS = 0.12) is dissolved in 100 g water while stirring. After homogenisation, 2.5 g TSTP is added to the solution and dissolved. Then, a solution of 5 g Span 65 in 100 g cyclohexane is added. After five minutes, a solution of 5.0 g NaOH in 50 g water is added. The reaction is performed while stirring at 450 rpm; the total reaction time is 4 hours. The reaction mixture is separated into two phases by addition of 100 ml acetic acid, and 250 ml water. The phase containing starch particles is removed and water is added to a total volume of 1000 ml. Subsequently, the starch particles are washed three times with 750 ml water in a separating funnel. After washing, the particles are preswollen in water for 18 hours, followed by precipitation in ethanol. The product has a water absorption capacity of 50 g/g after one hour, of which 98% is absorbed within the first minute. In a 0.9% NaCl solution, the absorption capacity is 18 g/g after one hour, of which 89% is absorbed within the first minute (or 91% within the first five minutes).

## Claims

1. A biodegradable, high water-absorbing polymer based on starch or derivatives thereof, characterised in that the starch, apart from optional cross-linking, has not been chemically modified or has been modified to a degree of substitution not exceeding 0.2, and has a water-absorbing power of at least 10 times its own weight, half of the water absorption being attained within 3 minutes.

2. A polymer according to claim 1, characterised in that the starch contains less than 1 ionic charge per 25 anhydroglucose units.

3. A polymer according to claim 1 or 2, characterised in that the starch has been crosslinked in a (quasi) co-continuous phase of a hydrophobic liquid and water.

4. A polymer according to claim 3, wherein the starch has been crosslinked with trisodium trimetaphosphate.

5. A process for producing a biodegradable, highly water-absorbing polymer based on starch or derivatives thereof, wherein starch, which has not been chemically modified or has been modified to a degree of substitution of less than 0.2, is crosslinked in a (quasi) co-continuous phase of a hydrophobic liquid and water.

6. A process according to claim 5, wherein 1-30 units of crosslinking agent are used per 100 anhydroglucose units.

7. A process according to claim 5 or 6, wherein the volume ratio of hydrophobic liquid and water is between 10:1 and 1:5.

8. A process according to any one of claims 5-7, wherein an emulsifier is used.

9. A process according to any one of claims 5-8, wherein the starch is at least partially gelatinised during or before crosslinking.

10. A process according to any one of claims 5-9, wherein the starch is preswollen after crosslinking.
